# EUROPEAN PATENT APPLICATION

(11) **EP 4 818 315 A1**
(43) Date of publication of application: **07.10.2026**
(21) Application number: 25907141.3
(22) Date of filing: 19.12.2025
(51) Int. Cl.: C12N 9/10, C12N 15/77, C12P 13/20, C12P 13/08, C12P 13/12, C12P 13/06

(54) **NOVEL TYPE II CITRATE SYNTHASE VARIANT AND METHOD FOR PRODUCING ASPARTATE-DERIVED L-AMINO ACIDS BY USING SAME**

(30) Priority: 20.12.2024 KR 20240193105
(71) Applicant: CJ Cheiljedang Corporation, Seoul 04560 (KR)
(72) Inventor: LEE, Hyein, Seoul 04560 (KR); KIM, Hye Mi, Seoul 04560 (KR); SON, Byung-Sam, Seoul 04560 (KR); SHIN, Kwang Soo, Seoul 04560 (KR); CHOE, Daegyu, Seoul 04560 (KR); JU, Siyeon, Seoul 04560 (KR); LEE, Jungseok, Seoul 04560 (KR)
(74) Representative: Meissner Bolte Partnerschaft mbB
(86) International application number: PCT/KR2025/022356
(87) International publication number: WO 2026/135368

(57) **Abstract**

The present disclosure relates to a type II citrate synthase variant, a *Corynebacterium glutamicum* strain comprising the type II citrate synthase variant, and a method for producing an aspartate-derived L-amino acid using the strain.

## Description

### [TECHNICAL FIELD]

### CROSS-REFERENCE TO RELATED APPLICATION(S)

The present disclosure claims the benefit of priority based on Korean Patent Application No. 10-2024-0193105 filed on December 20, 2024, and all contents disclosed in the documents of the corresponding Korean Patent Application are incorporated as part of the present disclosure.

A number of papers and patent documents are referenced and citations thereof are indicated throughout the present disclosure. The disclosure of the cited papers and patent documents is incorporated herein by reference in its entirety to more clearly explain the level of the technical field to which the present invention belongs and the content of the present invention.

The present disclosure relates to a novel type II citrate synthase variant, a *Corynebacterium glutamicum* strain comprising the type II citrate synthase variant, and a method for producing L-amino acid using the strain.

### [BACKGROUND ART]

In order to produce L-amino acids and other useful substances, various studies are being conducted for the development of high-efficiency production microorganisms and fermentation process technologies. For example, a target substance-specific approach, such as increasing the expression of a gene encoding an enzyme involved in L-lysine biosynthesis or removing genes unnecessary for biosynthesis, is mainly used (WO2008-082181 A1).

However, as the demand for L-amino acids increases, research for effectively increasing L-amino acid production ability is still needed.

### [CONTENTS OF THE INVENTION]

### [TECHNICAL PROBLEM]

One object of the present disclosure is to provide a type II citrate synthase variant.

Another object of the present disclosure is to provide a polynucleotide encoding the type II citrate synthase variant.

Still another object of the present disclosure is to provide a microorganism of the genus *Corynebacterium* comprising at least one selected from the group consisting of the type II citrate synthase variant and the polynucleotide encoding the same.

Still another object of the present disclosure is to provide a composition for producing an aspartate-derived L-amino acid comprising the microorganism of the genus *Corynebacterium*.

Still another object of the present disclosure is to provide a method for producing an aspartate-derived L-amino acid comprising a step of culturing in a medium the microorganism of the genus *Corynebacterium*.

Still another object of the present disclosure is to provide a use of the microorganism of the genus *Corynebacterium* for producing an L-amino acid.

### [TECHNICAL SOLUTION]

This will be explained in detail as follows. Meanwhile, each description and embodiment disclosed in the present application may also be applied to each other description and embodiment. That is, all combinations of the various elements disclosed in the present application fall within the scope of the present application. Furthermore, the scope of the present application should not be construed as being limited by the specific descriptions described below. In addition, a number of papers and patent documents are referenced throughout the present specification, and citations thereof are indicated. The disclosures of the cited papers and patent documents are incorporated herein by reference in their entirety to more clearly explain the level of the technical field to which the present invention belongs and the contents of the present invention.

One aspect of the present disclosure provides a type II citrate synthase variant, wherein an amino acid corresponding to position 272 from the N-terminus, an amino acid corresponding to position 310 from the N-terminus, or a combination thereof in the amino acid sequence of SEQ ID NO: 1 is substituted with another amino acid.

In the present disclosure, the term "type II citrate synthase" refers to an enzyme of the first step of the citric acid cycle that synthesizes citric acid by condensing acetyl-CoA and oxaloacetate derived from glycolysis or other catabolic reactions. Specifically, the type II citrate synthase of the present disclosure may be used interchangeably with the terms type II citrate synthase, GltA protein, or GltA. In the present disclosure, the sequence of the type II citrate synthase may be obtained from GenBank of NCBI, which is a known database; for example, it may be GenBank Accession No. WP_011013914.1. Specifically, it may be a polypeptide having type II citrate synthase activity encoded by the NCgl0795 gene (or *gltA* gene), but it is not limited thereto. In one embodiment, the type II citrate synthase may comprise the amino acid sequence of SEQ ID NO: 1 or may consist of the amino acid sequence of SEQ ID NO: 1, and as long as it has the same or corresponding conversion activity as the type II citrate synthase, a polypeptide having an amino acid sequence in which a partial sequence is deleted, modified, substituted, or added in the amino acid sequence of SEQ ID NO: 1 may also fall within the scope of the present disclosure. In addition, regardless of the origin of the microorganism, a polypeptide having sequence identity or homology of at least 60% or more, 70% or more, 75% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more with the amino acid sequence of SEQ ID NO: 1, and having the same or corresponding conversion activity as the type II citrate synthase, may also fall within the scope of the present disclosure as the type II citrate synthase.

In one embodiment, the type II citrate synthase variant of the present disclosure may have the activity of type II citrate synthase.

In one embodiment, the type II citrate synthase of the present disclosure may have weakened type II citrate synthase activity.

In the present disclosure, the sequence of SEQ ID NO: 1 is as follows:








 (SEQ ID NO: 1).

In one embodiment, the above-described type II citrate synthase variant of the present disclosure may comprise an amino acid sequence wherein an amino acid corresponding to position 272 from the N-terminus, an amino acid corresponding to position 310 from the N-terminus, or a combination thereof in the amino acid sequence of SEQ ID NO: 1 is substituted with another amino acid.

In one embodiment, the above-described type II citrate synthase variant of the present disclosure may essentially consist of an amino acid sequence wherein an amino acid corresponding to position 272 from the N-terminus, an amino acid corresponding to position 310 from the N-terminus, or a combination thereof in the amino acid sequence of SEQ ID NO: 1 is substituted with another amino acid.

In one embodiment, the above-described type II citrate synthase variant of the present disclosure may consist of an amino acid sequence wherein an amino acid corresponding to position 272 from the N-terminus, an amino acid corresponding to position 310 from the N-terminus, or a combination thereof in the amino acid sequence of SEQ ID NO: 1 is substituted with another amino acid.

In one embodiment, the amino acid corresponding to position 272 from the N-terminus in the amino acid sequence of SEQ ID NO: 1 may be leucine (Leu, L), but is not limited thereto.

In one embodiment, the amino acid corresponding to position 310 from the N-terminus in the amino acid sequence of SEQ ID NO: 1 may be arginine (Arg, R), but is not limited thereto.

In one embodiment, the above-described type II citrate synthase variant of the present disclosure may be a variant wherein the amino acid corresponding to position 272 from the N-terminus (e.g., leucine (Leu, L)) is substituted with glutamic acid (E), glycine (G), alanine (A), serine (S), threonine (T), cysteine (C), valine (V), isoleucine (I), methionine (M), proline (P), phenylalanine (F), tyrosine (Y), tryptophan (W), aspartic acid (D), glutamine (Q), histidine (H), lysine (K), arginine (R), or asparagine (N) (e.g., arginine (R));
the amino acid corresponding to position 310 from the N-terminus (e.g., arginine (Arg, R)) is substituted with glutamic acid (E), glycine (G), alanine (A), serine (S), threonine (T), cysteine (C), valine (V), leucine (L), isoleucine (I), methionine (M), proline (P), phenylalanine (F), tyrosine (Y), tryptophan (W), aspartic acid (D), glutamine (Q), histidine (H), lysine (K), or asparagine (N) (e.g., tyrosine (Y)); or
the amino acid corresponding to position 272 from the N-terminus (e.g., leucine (Leu, L)) is substituted with glutamic acid (E), glycine (G), alanine (A), serine (S), threonine (T), cysteine (C), valine (V), isoleucine (I), methionine (M), proline (P), phenylalanine (F), tyrosine (Y), tryptophan (W), aspartic acid (D), glutamine (Q), histidine (H), lysine (K), arginine (R), or asparagine (N) (e.g., arginine (R)), and the amino acid corresponding to position 310 from the N-terminus (e.g., arginine (Arg, R)) is substituted with glutamic acid (E), glycine (G), alanine (A), serine (S), threonine (T), cysteine (C), valine (V), leucine (L), isoleucine (I), methionine (M), proline (P), phenylalanine (F), tyrosine (Y), tryptophan (W), aspartic acid (D), glutamine (Q), histidine (H), lysine (K), or asparagine (N) (e.g., tyrosine (Y)), but is not limited thereto.

In one embodiment, the amino acid corresponding to position 272 from the N-terminus in the amino acid sequence of SEQ ID NO: 1 may be substituted with arginine (Arg, R), but is not limited thereto.

In one embodiment, the amino acid corresponding to position 310 from the N-terminus in the amino acid sequence of SEQ ID NO: 1 may be substituted with tyrosine (Tyr, Y), but is not limited thereto.

In one embodiment, the type II citrate synthase variant may be a variant wherein an amino acid corresponding to position 169 from the N-terminus, an amino acid corresponding to position 241 from the N-terminus, or a combination thereof in the amino acid sequence of SEQ ID NO: 1 is additionally substituted with another amino acid.

In one embodiment, the amino acid corresponding to position 169 from the N-terminus in the amino acid sequence of SEQ ID NO: 1 may be alanine (Ala, A), but is not limited thereto.

In one embodiment, the amino acid corresponding to position 241 from the N-terminus in the amino acid sequence of SEQ ID NO: 1 may be asparagine (Asn, N), but is not limited thereto.

In one embodiment, the amino acid corresponding to position 169 from the N-terminus may be substituted with glutamic acid (E), glycine (G), serine (S), threonine (T), cysteine (C), valine (V), leucine (L), isoleucine (I), methionine (M), proline (P), phenylalanine (F), tyrosine (Y), tryptophan (W), aspartic acid (D), glutamine (Q), histidine (H), lysine (K), arginine (R), or asparagine (N), and more specifically, may be substituted with valine (Val, V), but is not limited thereto.

In one embodiment, the amino acid corresponding to position 241 from the N-terminus may be substituted with glutamic acid (E), glycine (G), serine (S), threonine (T), cysteine (C), valine (V), leucine (L), isoleucine (I), methionine (M), proline (P), phenylalanine (F), tyrosine (Y), tryptophan (W), aspartic acid (D), glutamine (Q), histidine (H), lysine (K), arginine (R), or alanine (A), and more specifically, may be substituted with threonine (Thr, T), but is not limited thereto.

In the type II citrate synthase variant according to an embodiment of the present application, the amino acid corresponding to position 238 from the N-terminus in the amino acid sequence of SEQ ID NO: 1 may be an amino acid other than isoleucine (I, Ile), and more specifically, may be arginine (R), histidine (H), lysine (K), aspartic acid (D), glutamic acid (E), serine (S), threonine (T), asparagine (N), glutamine (Q), cysteine (C), glycine (G), proline (P), alanine (A), valine (V), leucine (L), methionine (M), phenylalanine (F), tyrosine (Y), or tryptophan (W), for example, may be histidine (His, H), but is not limited thereto.

In the type II citrate synthase variant according to an embodiment of the present application, the amino acid corresponding to position 393 from the N-terminus in the amino acid sequence of SEQ ID NO: 1 may be an amino acid other than tyrosine (Tyr, Y), and more specifically, may be arginine (R), histidine (H), lysine (K), aspartic acid (D), glutamic acid (E), serine (S), threonine (T), asparagine (N), glutamine (Q), cysteine (C), glycine (G), proline (P), alanine (A), valine (V), isoleucine (I), leucine (L), methionine (M), phenylalanine (F), or tryptophan (W), for example, may be phenylalanine (F), but is not limited thereto.

The type II citrate synthase variant of the present disclosure may comprise an amino acid sequence wherein an amino acid corresponding to position 272 from the N-terminus, an amino acid corresponding to position 310 from the N-terminus, or a combination thereof in the amino acid sequence of SEQ ID NO: 1 is substituted with another amino acid; or an amino acid sequence wherein, additionally thereto, an amino acid corresponding to position 169 from the N-terminus, an amino acid corresponding to position 241 from the N-terminus, or a combination thereof in the amino acid sequence of SEQ ID NO: 1 is substituted with another amino acid; or may comprise an amino acid sequence having homology or identity of at least 60%, 70%, 75%, 80%, 85%, 90%, 95%, 96%, 97%, 98%, 99%, 99.5%, or 99.7% or more with the amino acid sequence. In addition, it is obvious that any variant having an amino acid sequence in which a partial sequence is deleted, modified, substituted, conservatively substituted, or added also falls within the scope of the present disclosure, as long as it has such homology or identity and exhibits an effect corresponding to that of the variant of the present disclosure (an effect of increasing the production of an aspartate-derived L-amino acid (e.g., L-lysine)).

For example, this includes cases where there is a sequence addition or deletion, a naturally occurring mutation, a silent mutation, or a conservative substitution at the N-terminus, C-terminus, and/or within the amino acid sequence, which does not alter the function of the variant of the present disclosure.

In one embodiment, the above-described type II citrate synthase variant of the present disclosure may have homology or identity of at least 60%, 70%, 75%, 80%, 85%, 90%, 91%, 92%, 93%, 94%, 95%, 96%, 97%, 98%, 99%, 99.5% or 99.7% or more; and less than 100% with the amino acid sequence of SEQ ID NO: 1, but is not limited thereto.

In one embodiment, the above-described type II citrate synthase variant of the present disclosure may be a variant wherein an amino acid corresponding to position 272 from the N-terminus, an amino acid corresponding to position 310 from the N-terminus, or a combination thereof in the amino acid sequence of SEQ ID NO: 1 is substituted with another amino acid, having homology or identity of at least 60%, 70%, 75%, 80%, 85%, 90%, 91%, 92%, 93%, 94%, 95%, 96%, 97%, 98%, 99%, 99.5% or 99.7% or more; and less than 100% with the amino acid sequence of SEQ ID NO: 1, but is not limited thereto. In addition, it is obvious that any variant having an amino acid sequence in which a partial sequence is deleted, modified, substituted, conservatively substituted or added also falls within the scope of the present disclosure, as long as it includes the amino acid substitution as described above and has homology or identity as described above and exhibits an effect corresponding to the variant of the present application (an effect of increasing the production of an aspartate-derived L-amino acid (e.g., L-lysine)).The above-described type II citrate synthase variant of the present disclosure may be a variant wherein an amino acid corresponding to position 272 from the N-terminus, an amino acid corresponding to position 310 from the N-terminus, or a combination thereof in the amino acid sequence of SEQ ID NO: 1 is substituted with another amino acid, having homology or identity of at least 60%, 70%, 75%, 80%, 85%, 90%, 91%, 92%, 93%, 94%, 95%, 96%, 97%, 98%, 99%, 99.5% or 99.7% or more, and less than 100% with the amino acid sequence of SEQ ID NO: 1, and exhibiting type II citrate synthase activity, but is not limited thereto.

In one embodiment, the above-described type II citrate synthase variant of the present disclosure may be a variant wherein an amino acid corresponding to position 272 from the N-terminus, an amino acid corresponding to position 310 from the N-terminus, or a combination thereof in the amino acid sequence of SEQ ID NO: 1 is substituted with another amino acid, and additionally an amino acid corresponding to position 169 from the N-terminus, an amino acid corresponding to position 241 from the N-terminus, or a combination thereof in the amino acid sequence of SEQ ID NO: 1 is substituted with another amino acid, having homology or identity of at least 60%, 70%, 75%, 80%, 85%, 90%, 91%, 92%, 93%, 94%, 95%, 96%, 97%, 98%, 99%, 99.5% or 99.7% or more; and less than 100% with the amino acid sequence of SEQ ID NO: 1, but is not limited thereto. In addition, it is obvious that any variant having an amino acid sequence in which a partial sequence is deleted, modified, substituted, conservatively substituted or added is also falls within the scope of the present disclosure, as long as it includes the amino acid substitution as described above and has homology or identity as described above and exhibits an effect corresponding to the variant of the present application (an effect of increasing the production of an aspartate-derived L-amino acid (e.g., L-lysine))..

The above-described type II citrate synthase variant of the present disclosure may be a variant wherein an amino acid corresponding to position 272 from the N-terminus, an amino acid corresponding to position 310 from the N-terminus, or a combination thereof in the amino acid sequence of SEQ ID NO: 1 is substituted with another amino acid, and additionally an amino acid corresponding to position 169 from the N-terminus, an amino acid corresponding to position 241 from the N-terminus, or a combination thereof in the amino acid sequence of SEQ ID NO: 1 is substituted with another amino acid, having homology or identity of at least 60%, 70%, 75%, 80%, 85%, 90%, 91%, 92%, 93%, 94%, 95%, 96%, 97%, 98%, 99%, 99.5% or 99.7% or more, and less than 100% with the amino acid sequence of SEQ ID NO: 1, and exhibiting type II citrate synthase activity, but is not limited thereto.

In one embodiment, the above-described type II citrate synthase variant of the present disclosure may comprise the amino acid sequence of SEQ ID NO: 3, SEQ ID NO: 4, SEQ ID NO: 9 or SEQ ID NO: 10. In another embodiment, the type II citrate synthase variant may essentially consist of the amino acid sequence of SEQ ID NO: 3, SEQ ID NO: 4, SEQ ID NO: 9 or SEQ ID NO: 10. In still another embodiment, the type II citrate synthase variant may consist of the amino acid sequence of SEQ ID NO: 3, SEQ ID NO: 4, SEQ ID NO: 9 or SEQ ID NO: 10.

The type II citrate synthase variant of the present disclosure may comprise the amino acid sequence of SEQ ID NO: 3, SEQ ID NO: 4, SEQ ID NO: 9 or SEQ ID NO: 10, or may comprise an amino acid sequence having homology or identity of at least 60%, 70%, 75%, 80%, 85%, 90%, 95%, 96%, 97%, 98%, 99%, 99.5%, 99.7% or 99.9% or more with the amino acid sequence. In addition, it is obvious that a variant having an amino acid sequence in which a partial sequence is deleted, modified, substituted, conservatively substituted or added also falls within the scope of the present disclosure, as long as it has such homology or identity and exhibits an effect corresponding to the variant of the present application (an effect of increasing the production of an aspartate-derived L-amino acid (e.g., L-lysine))..

For example, this includes cases where there is a sequence addition or deletion, a naturally occurring mutation, a silent mutation, or a conservative substitution at the N-terminus, C-terminus, and/or within the amino acid sequence, which does not alter the function of the variant of the present disclosure.

In one embodiment, the type II citrate synthase variant of the present disclosure may comprise the amino acid sequence of SEQ ID NO: 3, SEQ ID NO: 4, SEQ ID NO: 9 or SEQ ID NO: 10, and may have homology or identity of at least 60%, 70%, 75%, 80%, 85%, 90%, 91%, 92%, 93%, 94%, 95%, 96%, 97%, 98%, 99%, 99.5% or 99.7% or more; and less than 100% with the amino acid sequence of SEQ ID NO: 1, but is not limited thereto. In addition, it is obvious that a variant having an amino acid sequence in which a partial sequence is deleted, modified, substituted, conservatively substituted or added also falls within the scope of the present disclosure, as long as it has such homology or identity and exhibits an effect corresponding to the variant of the present application (an effect of increasing the production of an aspartate-derived L-amino acid (e.g., L-lysine)).

The above-described type II citrate synthase variant of the present disclosure may comprise the amino acid sequence of SEQ ID NO: 3, SEQ ID NO: 4, SEQ ID NO: 9 or SEQ ID NO: 10, and may have homology or identity of at least 60%, 70%, 75%, 80%, 85%, 90%, 91%, 92%, 93%, 94%, 95%, 96%, 97%, 98%, 99%, 99.5% or 99.7% or more; and less than 100% with the amino acid sequence of SEQ ID NO: 1, and exhibiting type II citrate synthase activity, but is not limited thereto.

The "conservative substitution" refers to substituting one amino acid with another amino acid having similar structural and/or chemical properties. Such amino acid substitution may generally occur based on similarities in polarity, charge, solubility, hydrophobicity, hydrophilicity and/or amphipathic nature of the residues. Typically, conservative substitution may have little or no effect on the activity of a protein or polypeptide.

In the present disclosure, the term "variant" refers to a polypeptide in which one or more amino acids are conservatively substituted and/or modified, so that it is different from the amino acid sequence before variation of the variant, but functions or properties are maintained. Such variants can generally be identified by modifying one or more amino acids in the amino acid sequence of the polypeptide and evaluating the properties of the modified polypeptide. That is, the ability of the variant may be increased, unchanged, or decreased compared to the polypeptide before variation. In addition, some variants may include variants in which one or more parts such as an N-terminal leader sequence or a transmembrane domain are removed. Other variants may include variants in which a portion is removed from the N- and/or C-terminus of a mature protein. The term "variant" may be used interchangeably with terms such as variant form, modification, variant polypeptide, modified protein, mutant, and divergent (as English expressions, modification, modified polypeptide, modified protein, mutant, mutein, divergent, and the like), and is not limited thereto as long as it is used to mean a mutated form.

In addition, the variant may include deletion or addition of amino acids having minimal influence on properties and secondary structure of the polypeptide. For example, a signal (or leader) sequence involved in translocation of a protein co-translationally or post-translationally may be conjugated to the N-terminus of the variant. In addition, the variant may be conjugated with another sequence or linker for identification, purification, or synthesis.

In the present disclosure, the terms 'homology' or 'identity' refer to the degree of similarity between two given amino acid sequences or nucleotide sequences and can be expressed as a percentage. The terms homology and identity can often be used interchangeably.

The sequence homology or identity of a conserved polynucleotide or polypeptide is determined by a standard alignment algorithm, and a default gap penalty established by the program used may be used together. Substantially, homologous or identical sequences can generally hybridize with all or part of the sequence under moderately or highly stringent conditions. It is obvious that hybridization also includes hybridization with a polynucleotide containing standard codons or codons taking into account codon degeneracy in the polynucleotide.

Whether any two polynucleotide or polypeptide sequences have homology or identity can be determined using a known computer algorithm such as the "FASTA" program using default parameters as in, for example, Pearson et al (1988) [Proc. Natl. Acad. Sci. USA 85]: 2444. Alternatively, it can be determined using the Needleman-Wunsch algorithm (Needleman and Wunsch, 1970, J. Mol. Biol. 48: 443-453) as performed in the Needleman program (EMBOSS: The European Molecular Biology Open Software Suite, Rice et al., 2000, Trends Genet. 16: 276-277) (version 5.0.0 or later) of the EMBOSS package (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego, 1994, and [CARILLO ETA/.] (1988) SIAM J Applied Math 48: 1073). For example, homology or identity can be determined using BLAST of the National Center for Biotechnology Information or ClustalW.

The homology or identity of a polynucleotide or polypeptide can be determined by comparing sequence information using a GAP computer program such as, for example, Needleman et al. (1970), J Mol Biol. 48:443, as disclosed in Smith and Waterman, Adv. Appl. Math (1981) 2:482. In summary, the GAP program can be defined as a value obtained by dividing the number of similar aligned symbols (i.e., nucleotides or amino acids) by the total number of symbols in the shorter of the two sequences. Default parameters for the GAP program include (1) a binary comparison matrix (containing a value of 1 for identity and 0 for non-identity) and a weighted comparison matrix of Gribskov et al (1986) Nucl. Acids Res. 14: 6745 (or EDNAFULL (EMBOSS version of NCBI NUC4.4) substitution matrix) as disclosed by Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or a gap opening penalty of 10, a gap extension penalty of 0.5); and (3) no penalty for terminal gaps.

As an embodiment of the present disclosure, the variant of the present disclosure may have an activity to increase aspartate-derived L-amino acid (e.g., L-lysine) production ability compared to wild-type type II citrate synthase.

In the present application, the term "aspartate (Aspartic acid)" is refers to an α-amino acid used for protein biosynthesis, can be used interchangeably with "aspartic acid", is abbreviated as Asp or D, and includes an amino group and a carboxylic acid like all other amino acids. Generally, aspartate may be produced as aspartyl phosphate by aspartokinase (LysC) and then may be converted into L-lysine, L-threonine, L-methionine, L-homoserine, or L-isoleucine in vivo.

In the present disclosure, the term "aspartate-derived L-amino acid" refers to a substance that can be biosynthesized using aspartate (aspartic acid) as a precursor, and is not limited as long as it is a substance that can be produced through a biosynthesis process using aspartate as a precursor. The aspartate-derived L-amino acid may include not only the aspartate-derived L-amino acid itself but also derivatives thereof. For example, it may be L-lysine, L-threonine, L-methionine, L-glycine, homoserine, O-acetylhomoserine, O-succinylhomoserine, O-phosphohomoserine, L-isoleucine, and/or cadaverine, but is not limited thereto. In one embodiment, the aspartate-derived L-amino acid may be one or more selected from the group consisting of L-aspartate, L-lysine, L-threonine, L-methionine, L-homoserine, and L-isoleucine, and more specifically, may be L-lysine, but is not limited thereto.

In the present disclosure, the term "corresponding to" refers to an amino acid residue at a position listed in a polypeptide, or an amino acid residue similar, identical, or homologous to a residue listed in a polypeptide. Identifying an amino acid at a corresponding position may involve determining a specific amino acid in a sequence with reference to a specific reference sequence. As used in the present disclosure, "corresponding region" generally refers to a similar or corresponding position in a related protein or reference protein.

For example, a given amino acid sequence may be aligned with: an amino acid sequence wherein an amino acid corresponding to position 272 from the N-terminus, an amino acid corresponding to position 310 from the N-terminus, or a combination thereof in the amino acid sequence of SEQ ID NO: 1 is substituted with another amino acid; an amino acid sequence wherein, additionally thereto, an amino acid corresponding to position 169 from the N-terminus, an amino acid corresponding to position 241 from the N-terminus, or a combination thereof in the amino acid sequence of SEQ ID NO: 1 is further substituted with another amino acid; or the amino acid sequence of SEQ ID NO: 3, SEQ ID NO: 4, SEQ ID NO: 9 or SEQ ID NO: 10, and based on this alignment, each amino acid residue of the given amino acid sequence can be numbered with reference to the numerical position of the corresponding amino acid residue of: amino acid sequence wherein an amino acid corresponding to position 272 from the N-terminus, an amino acid corresponding to position 310 from the N-terminus, or a combination thereof in the amino acid sequence of SEQ ID NO: 1 is substituted with another amino acid; or the amino acid sequence wherein, additionally thereto, an amino acid corresponding to position 169 from the N-terminus, an amino acid corresponding to position 241 from the N-terminus, or a combination thereof in the amino acid sequence of SEQ ID NO: 1 is further substituted with another amino acid; or the amino acid sequence of SEQ ID NO: 3, SEQ ID NO: 4, SEQ ID NO: 9 or SEQ ID NO: 10. For example, a sequence alignment algorithm such as that described in the present disclosure can identify the position of an amino acid, or a position where a modification such as substitution, insertion, or deletion occurs, in comparison with a query sequence (also referred to as a "reference sequence").

For such alignment, for example, the Needleman-Wunsch algorithm (Needleman and Wunsch, 1970, J. Mol. Biol. 48: 443-453), the Needle program (EMBOSS: The European Molecular Biology Open Software Suite, Rice et al., 2000, Trends Genet. 16: 276-277) of the EMBOSS package, and the like may be used, but is not limited thereto, and a sequence alignment program, a pairwise sequence comparison algorithm, and the like known in the art may be appropriately used.

Another aspect of the present disclosure is to provide the polynucleotide encoding the type II citrate synthase variant of the present disclosure.

In the present disclosure, the term "polynucleotide" refers to a polymer of nucleotides in which nucleotide monomers are linked in a long chain by covalent bonds, and refers to a DNA or RNA strand of a certain length or greater, and more specifically, refers to a polynucleotide fragment encoding the variant.

In one embodiment, the polynucleotide may be a polynucleotide encoding the type II citrate synthase variant comprising an amino acid sequence wherein an amino acid corresponding to position 272 from the N-terminus, an amino acid corresponding to position 310 from the N-terminus, or a combination thereof in the amino acid sequence of SEQ ID NO: 1 is substituted with another amino acid; or an amino acid sequence wherein, in addition thereto, an amino acid corresponding to position 169 from the N-terminus, an amino acid corresponding to position 241 from the N-terminus, or a combination thereof in the amino acid sequence of SEQ ID NO: 1 is substituted with another amino acid; or the amino acid sequence of SEQ ID NO: 3, SEQ ID NO: 4, SEQ ID NO: 9, or SEQ ID NO: 10.

In one embodiment, the polynucleotide may comprise any one nucleotide sequence selected from the group consisting of the nucleotide sequences of SEQ ID NO: 11, SEQ ID NO: 12, SEQ ID NO: 17, and SEQ ID NO: 18.

The polynucleotide encoding the type II citrate synthase variant of the present disclosure may have or comprise any one nucleotide sequence selected from the group consisting of the nucleotide sequences of SEQ ID NO: 11, SEQ ID NO: 12, SEQ ID NO: 17, and SEQ ID NO: 18. In another embodiment, the polynucleotide may comprise a nucleotide sequence having homology or identity of at least 60%, 70%, 75%, 80%, 85%, 90%, 95%, 96%, 97%, 98%, 99%, 99.5%, 99.7% or 99.9% or more with the nucleotide sequence. In addition, it is obvious that a polynucleotide having a nucleotide sequence in which a partial sequence is deleted, modified, substituted, conservatively substituted or added also falls within the scope of the present disclosure, as long as it has such homology or identity and encodes a polypeptide or protein that exhibits an effect corresponding to the variant of the present disclosure (an effect of increasing the production of an aspartate-derived L-amino acid (e.g., L-lysine)).

In one embodiment, the nucleotide sequence or amino acid sequence provided herein may include those modified by conventional mutagenesis methods such as directed evolution and/or site-directed mutagenesis, within a range that maintains their original function or desired function. In one embodiment, a polynucleotide or polypeptide "comprising a specific nucleotide sequence or amino acid sequence" may mean that the polynucleotide or polypeptide (i) consists of or essentially comprises the specific nucleotide sequence or amino acid sequence, or (ii) consists of or essentially comprises an amino acid sequence having homology of 60% or more, 70% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more with the specific nucleotide sequence or amino acid sequence and maintains the original function and/or desired function.

The polynucleotide of the present disclosure may include various modifications in the coding region within a range that does not alter the amino acid sequence of the variant of the present disclosure, in consideration of codon degeneracy or codons preferred in an organism intended to express the variant of the present disclosure. Specifically, the polynucleotide of the present disclosure may have or comprise a nucleotide sequence having homology or identity of 60% or more, 70% or more, 75% or more, 80% or more, 85% or more, 90% or more, 95% or more, 96% or more, 97% or more, 98% or more, and less than 100% with any one nucleotide sequence selected from the group consisting of:
a nucleotide sequence of the polynucleotide encoding the type II citrate synthase variant comprising: an amino acid sequence wherein an amino acid corresponding to position 272 from the N-terminus, an amino acid corresponding to position 310 from the N-terminus, or a combination thereof in the amino acid sequence of SEQ ID NO: 1 is substituted with another amino acid; an amino acid sequence wherein, additionally thereto, an amino acid corresponding to position 169 from the N-terminus, an amino acid corresponding to position 241 from the N-terminus, or a combination thereof in the amino acid sequence of SEQ ID NO: 1 is further substituted with another amino acid; or the amino acid sequence of SEQ ID NO: 3, SEQ ID NO: 4, SEQ ID NO: 9 or SEQ ID NO: 10, and
the nucleotide sequences of SEQ ID NO: 11, SEQ ID NO: 12, SEQ ID NO: 17, and SEQ ID NO: 18;
or the polynucleotide may consist of or essentially consist of a nucleotide sequence having homology or identity of 60% or more, 70% or more, 75% or more, 80% or more, 85% or more, 90% or more, 95% or more, 96% or more, 97% or more, 98% or more, and less than 100% with the nucleotide sequence, but is not limited thereto.

In addition, the polynucleotide of the present disclosure may include, without limitation, any sequence that can hybridize under stringent conditions with a probe prepared from a known gene sequence, for example, a sequence complementary to all or part of the polynucleotide sequence of the present disclosure. The "stringent conditions" refer to conditions that enable specific hybridization between polynucleotides. For example, the conditions may include: conditions under which polynucleotides with high homology or identity, such as polynucleotides having homology or identity of 70% or more, 75% or more, 80% or more, 85% or more, 90% or more, 95% or more, 96% or more, 97% or more, 98% or more, or 99% or more, hybridize with each other, while polynucleotides with lower homology or identity do not hybridize with each other; or conditions under which washing is performed once, specifically 2 to 3 times, at a salt concentration and temperature corresponding to washing conditions of conventional Southern hybridization, which are 60°C, 1×SSC, 0.1% SDS, specifically 60°C, 0.1×SSC, 0.1% SDS, and more specifically 68°C, 0.1×SSC, 0.1% SDS.

Hybridization requires that two nucleic acids have complementary sequences, although mismatches between bases are possible depending on the stringency of hybridization. The term "complementary" is used to describe the relationship between nucleotide bases that are capable of hybridizing with each other. For example, with respect to DNA, adenine is complementary to thymine and cytosine is complementary to guanine. Therefore, the polynucleotide of the present disclosure may also include isolated nucleic acid fragments that are complementary to the entire sequence as well as substantially similar nucleotide sequences.

Specifically, a polynucleotide having homology or identity with the polynucleotide of the present disclosure can be detected using hybridization conditions including a hybridization step at a Tm value of 55°C and using the above-described conditions. In addition, the Tm value may be 60°C, 63°C or 65°C, but is not limited thereto, and may be appropriately adjusted by those skilled in the art according to the purpose.

The appropriate stringency for hybridizing the polynucleotide depends on the length and degree of complementarity of the polynucleotide, and such parameters are well known in the art.

Another aspect of the present disclosure is to provide a vector comprising the polynucleotide of the present disclosure. The vector may be an expression vector for expressing the polynucleotide in a host cell, but is not limited thereto.

The vector of the present disclosure may comprise a DNA construct comprising a nucleotide sequence of the polynucleotide encoding a target polypeptide operably linked to a suitable expression control region (or expression control sequence) so that the target polypeptide can be expressed in a suitable host. The expression control region may comprise a promoter capable of initiating transcription, any operator sequence for regulating such transcription, a suitable sequence encoding a ribosome binding site, and a sequence regulating the termination of transcription and translation. After the vector is transformed into a suitable host cell, it can be replicated or function independently of the host genome, or can be integrated into the genome itself.

The vector used in the present disclosure is not particularly limited, and any vector known in the art can be used. Examples of commonly used vectors include plasmids, cosmids, viruses, and bacteriophages in a natural or recombinant state. For example, pWE15, M13, MBL3, MBL4, IXII, ASHII, APII, t10, t11, Charon4A, Charon21A, and the like can be used as phage vectors or cosmid vectors, and pDZ series, pBR series, pUC series, pBluescriptII series, pGEM series, pTZ series, pCL series, pET series, and the like can be used as plasmid vectors. Specifically, pDZ, pDC, pDCM2, pACYC177, pACYC184, pCL, pECCG117, pUC19, pBR322, pMW118, pCC1BAC, or pDC24 vector, among others, can be used.

For example, a polynucleotide encoding a target polypeptide can be inserted into a chromosome through a vector for chromosomal insertion in a cell. Insertion of the polynucleotide into the chromosome may be achieved by any method known in the art, for example, homologous recombination, but is not limited thereto. A selection marker for confirming the chromosomal insertion may be further included. The selection marker is for selecting cells transformed with the vector, that is, for confirming whether the target nucleic acid molecule is inserted, and markers that confer a selectable phenotype such as drug resistance, auxotrophy, resistance to cytotoxic agents, or expression of a surface polypeptide can be used. In an environment treated with a selective agent, only cells expressing the selection marker survive or exhibit other phenotypes, so that transformed cells can be selected.

In the present disclosure, the term "transformation" means introducing a vector comprising a polynucleotide encoding a target polypeptide into a host cell or microorganism so that the polypeptide encoded by the polynucleotide may be expressed in the host cell. The transformed polynucleotide may be inserted and located in the chromosome of the host cell or located outside the chromosome, as long as it may be expressed in the host cell, and includes all of these. In addition, the polynucleotide comprises DNA and/or RNA encoding the target polypeptide. The polynucleotide may be introduced in any form as long as it may be introduced into and expressed in a host cell. For example, the polynucleotide may be introduced into a host cell in the form of an expression cassette, which is a gene construct comprising all elements required for its own expression. The expression cassette usually may comprise a promoter, a transcription termination signal, a ribosome binding site, and a translation termination signal operably linked to the polynucleotide. The expression cassette may be in the form of a self-replicable expression vector. In addition, the polynucleotide may be introduced into the host cell in its own form and be operably linked to a sequence required for expression in the host cell, but is not limited thereto..

In addition, in the above, the term "operably linked" means that a promoter sequence is functionally linked to the polynucleotide sequence to initiate and mediate transcription of the polynucleotide encoding the target variant of the present disclosure.

Another aspect of the present disclosure provides a microorganism of the genus *Corynebacterium* comprising at least one selected from the group consisting of the type II citrate synthase variant of the present disclosure and the polynucleotide of the present disclosure.

The microorganism of the present disclosure may comprise the type II citrate synthase variant of the present disclosure, a polynucleotide encoding the type II citrate synthase variant, or a vector comprising the polynucleotide of the present disclosure.

In the microorganism, the polynucleotide encoding the type II citrate synthase variant may exist or be located in a modified or mutated form of the nucleotide sequence encoding an existing (pre-mutation or wild-type) type II citrate synthase on the chromosome of the microorganism, or may include a case where it is exogenously introduced. When exogenously introduced, the polynucleotide encoding the type II citrate synthase variant may be inserted into a target position through homologous recombination or the like, inserted into a non-target position by a non-homologous mechanism, or exist in the form of a vector such as a plasmid, but is not limited thereto.

In the present disclosure, the term "microorganism (or, strain)" includes both wild-type microorganisms and microorganisms in which genetic modification has occurred naturally or artificially, and is a microorganism in which a specific mechanism is weakened or enhanced due to causes such as insertion of an external gene or enhancement or inactivation of the activity of an endogenous gene, and may be a microorganism comprising genetic modification for the production of a target polypeptide, protein, or product.

The microorganism of the present disclosure may be a microorganism comprising at least one of the type II citrate synthase variant of the present disclosure, the polynucleotide of the present disclosure, and the vector comprising the polynucleotide of the present disclosure; a microorganism modified to express the variant of the present disclosure or the polynucleotide of the present disclosure; a microorganism (e.g., a recombinant microorganism) expressing the variant of the present disclosure or the polynucleotide of the present disclosure; or a microorganism (e.g., a recombinant microorganism) having the variant activity of the present disclosure, but is not limited thereto.

The microorganism of the present disclosure may be a microorganism having aspartate-derived L-amino acid (e.g., L-lysine) production ability.

The aspartate-derived L-amino acid is as described above.

The microorganism of the present disclosure may be a microorganism naturally having aspartate-derived L-amino acid (e.g., L-lysine) production ability, or a microorganism in which the variant of the present disclosure or a polynucleotide encoding the same (or a vector comprising the polynucleotide) is introduced into a parent strain lacking aspartate-derived L-amino acid (e.g., L-lysine) production ability and/or a microorganism to which aspartate-derived L-amino acid (e.g., L-lysine) production ability is conferred, but is not limited thereto.

In one embodiment, the microorganism of the present disclosure may be a cell or microorganism transformed with the polynucleotide of the present disclosure or a vector comprising a polynucleotide encoding the variant of the present disclosure to express the type II citrate synthase variant of the present disclosure, and for the purpose of the present disclosure, the strain of the present disclosure may include any microorganism that comprises the variant of the present disclosure and is capable of producing an aspartate-derived L-amino acid (e.g., L-lysine). For example, the microorganism of the present disclosure may be a recombinant microorganism with increased aspartate-derived L-amino acid (e.g., L-lysine) production ability by introducing a polynucleotide encoding the variant of the present disclosure into a natural wild-type microorganism or a microorganism producing an aspartate-derived L-amino acid (e.g., L-lysine).

In one embodiment, the microorganism of the present disclosure may have an increased production ability of an aspartate-derived L-amino acid (e.g., L-lysine), compared to a microorganism of the genus *Corynebacterium* that does not comprise any one selected from the group consisting of the above-described type II citrate synthase variant of the present disclosure and the polynucleotide encoding the type II citrate synthase variant (which is a microorganism not expressing a type II citrate synthase variant comprising an amino acid sequence wherein an amino acid corresponding to position 272 from the N-terminus, an amino acid corresponding to position 310 from the N-terminus, or a combination thereof in the amino acid sequence of SEQ ID NO: 1 is substituted with another amino acid; or an amino acid sequence wherein, additionally thereto, an amino acid corresponding to position 169 from the N-terminus, an amino acid corresponding to position 241 from the N-terminus, or a combination thereof in the amino acid sequence of SEQ ID NO: 1 is substituted with another amino acid; or the amino acid sequence of SEQ ID NO: 3, SEQ ID NO: 4, SEQ ID NO: 9 or SEQ ID NO: 10, or a microorganism expressing a wild-type protein corresponding to the type II citrate synthase variant).

The microorganism of the genus *Corynebacterium* not comprising any one selected from the group consisting of the type II citrate synthase variant of the present disclosure and the polynucleotide encoding the type II citrate synthase variant may be a natural wild-type microorganism or an unmodified microorganism, and may also be expressed as a host cell or a parent strain. The microorganism of the genus *Corynebacterium* not comprising any one selected from the group consisting of the type II citrate synthase variant of the present disclosure and the polynucleotide encoding the type II citrate synthase variant may specifically be ATCC13032 strain and/or CJ3P (US 9556463 B2), but is not limited thereto.

The recombinant microorganism with increased aspartate-derived L-amino acid (e.g., L-lysine) production ability of the present disclosure may be a microorganism with increased production ability compared to a natural wild-type microorganism or an unmodified microorganism, but is not limited thereto. As an example, the unmodified microorganism, which is a target strain for comparing whether the aspartate-derived L-amino acid (e.g., L-lysine) production ability is increased, may be ATCC13032 strain and/or CJ3P (US 9556463 B2), but is not limited thereto.

In one embodiment, the microorganism with increased aspartate-derived L-amino acid (e.g., L-lysine) production ability (production amount) of the present disclosure may have increased aspartate-derived L-amino acid (e.g., L-lysine) production ability (production amount) by about 1% or more, about 5% or more, about 10% or more, about 15% or more, about 20% or more, or about 21% or more (the upper limit is not particularly limited, and for example, may be about 200% or less, about 150% or less, about 100% or less, or about 50% or less, or about 30% or less) compared to a parent strain before mutation or an unmodified microorganism, but is not limited thereto. In another embodiment, the microorganism with increased aspartate-derived L-amino acid (e.g., L-lysine) production ability (production amount) of the present disclosure may have increased aspartate-derived L-amino acid (e.g., L-lysine) production ability (production amount) by about 1.01-fold or more, about 1.05-fold or more, about 1.1-fold or more, about 1.15-fold or more, about 1.2-fold or more, or about 1.21-fold or more (the upper limit is not particularly limited, and for example, may be about 10-fold or less, about 5-fold or less, about 3-fold or less, or about 2-fold or less) compared to a parent strain before mutation or an unmodified microorganism, but is not limited thereto.

The term "about" refers to a range including all values of ±0.5, ±0.4, ±0.3, ±0.2, ±0.1, and the like, and includes all numerical values in a range equivalent or similar to the numerical value following the term "about", but is not limited thereto.

In the present disclosure, the term "unmodified microorganism" does not exclude a microorganism containing a naturally occurring mutation in a microorganism, and may mean a wild-type microorganism or a natural-type microorganism itself, or a microorganism before a trait is changed by genetic variation due to natural or artificial factors. For example, the unmodified microorganism may mean a microorganism into which the type II citrate synthase variant described in the present specification is not introduced or before it is introduced. The "unmodified microorganism" may be used interchangeably with "strain before modification", "microorganism before modification", "non-mutated strain", "unmodified strain", "non-mutated microorganism" or "reference microorganism".

In one embodiment, the microorganism of the present disclosure may be *Corynebacterium glutamicum*, *Corynebacterium crudilactis*, *Corynebacterium deserti*, *Corynebacterium efficiens*, *Corynebacterium callunae*, *Corynebacterium stationis*, *Corynebacterium singulare*, *Corynebacterium halotolerans*, *Corynebacterium striatum*, *Corynebacterium ammoniagenes*, *Corynebacterium pollutisoli*, *Corynebacterium imitans*, *Corynebacterium testudinoris*, or *Corynebacterium flavescens*, and more specifically, may be *Corynebacterium glutamicum*.

In the present disclosure, the term "weakening" of a polypeptide is a concept that includes both a decrease in activity and a lack of activity, as compared to the intrinsic activity. The term weakening can be used interchangeably with terms such as inactivation, deficiency, down-regulation, decrease, reduction, and attenuation.

The weakening may include a case where the activity of the polypeptide itself is reduced or removed compared to the activity of the polypeptide originally possessed by the microorganism due to mutation of the polynucleotide encoding the polypeptide or the like, a case where the overall degree of polypeptide activity and/or concentration (expression level) in the cell is lower than that of the natural strain due to inhibition of expression of a gene of the encoding polynucleotide or inhibition of translation into a polypeptide, a case where the polynucleotide is not expressed at all, and/or a case where there is no activity of the polypeptide even if the polynucleotide is expressed. The "intrinsic activity" refers to the activity of a specific polypeptide originally possessed by a parent strain, wild-type, or unmodified microorganism before its trait is changed due to genetic variation caused by natural or artificial factors. This can be used interchangeably with "activity before modification". That the activity of a polypeptide is "inactivated, deficient, decreased, down-regulated, reduced, or attenuated" compared to the intrinsic activity means that it has become lower than the activity of the specific polypeptide originally possessed by the parent strain or unmodified microorganism before the trait change.

Such weakening of polypeptide activity can be performed by any method known in the art, but is not limited thereto, and can be achieved by application of various methods well known in the art (e.g., Nakashima N et al., Bacterial cellular engineering by genome editing and gene silencing. Int J Mol Sci. 2014;15(2):2773-2793, Sambrook et al. Molecular Cloning 2012, and the like).

Specifically, the weakening of a polypeptide of the present disclosure may be by
1) deletion of all or part of the gene encoding the polypeptide;
2) modification of an expression control region (or expression control sequence) so that expression of a gene encoding the polypeptide decreases;
3) modification of amino acid sequence constituting the polypeptide so that the activity of the polypeptide is removed or weakened (e.g., deletion/substitution/addition of one or more amino acids on the amino acid sequence);
4) modification of the gene sequence encoding the polypeptide so that the activity of the polypeptide is removed or weakened (for example, deletion/substitution/addition of one or more nucleic acid bases on the nucleic acid base sequence of the polypeptide gene so as to code for a polypeptide modified so that the activity of the polypeptide is removed or weakened);
5) modification of an initiation codon of a gene transcript encoding the polypeptide or a nucleotide sequence encoding a 5'-UTR region;
6) introduction of an antisense oligonucleotide (e.g., antisense RNA) that complementarily binds to the transcript of the gene encoding the polypeptide;
7) addition of a sequence complementary to a Shine-Dalgarno sequence to the front of the Shine-Dalgarno sequence of the gene encoding the polypeptide to form a secondary structure incapable of ribosome attachment;
8) addition of a promoter transcribed in the reverse direction to the 3' terminus of an open reading frame (ORF) of the gene sequence encoding the polypeptide (Reverse transcription engineering, RTE); or
9) regulation of cellular localization of a protein (polypeptide) (cellular localization); or
10) a combination of two or more selected from the above 1) to 9), but is not particularly limited thereto.

For example, the 1) deletion of part or all of the gene encoding the polypeptide may be removal of the entire endogenous polynucleotide encoding a target polypeptide in the chromosome, replacement with a polynucleotide in which some nucleotides are deleted, or replacement with a marker gene.

In addition, the 2) modification of the expression control region (or expression control sequence) may be generation of a mutation on the expression control region (or expression control sequence) by deletion, insertion, non-conservative or conservative substitution, or a combination thereof, or replacement with a sequence having weaker activity. The expression control region includes, but is not limited to, a promoter, an operator sequence, a sequence encoding a ribosome binding site, and a sequence regulating the termination of transcription and translation.

In addition, the 3) modification of an initiation codon of a gene transcript encoding the polypeptide or a nucleotide sequence encoding a 5'-UTR region may be, for example, substituting with a nucleotide sequence encoding another initiation codon having a lower polypeptide expression rate compared to the endogenous initiation codon, but is not limited thereto.

In addition, the modification of the amino acid sequence or polynucleotide sequence in 4) and 5) may be generation of a mutation on the sequence by deletion, insertion, non-conservative or conservative substitution, or a combination thereof in the amino acid sequence of the polypeptide or the polynucleotide sequence encoding the polypeptide so as to weaken the activity of the polypeptide, or replacement with an amino acid sequence or polynucleotide sequence improved to have weaker activity or an amino acid sequence or polynucleotide sequence improved to have no activity, but is not limited thereto. For example, by introducing a mutation in the polynucleotide sequence to form a stop codon, expression of a gene can be inhibited or weakened, but is not limited thereto.

For the 6) introduction of an antisense oligonucleotide (e.g., antisense RNA) that complementarily binds to the transcript of the gene encoding the polypeptide, for example, reference can be made to [Weintraub, H. et al., Antisense-RNA as a molecular tool for genetic analysis, Reviews - Trends in Genetics, Vol. 1(1) 1986].

The 7) addition of a sequence complementary to a Shine-Dalgarno sequence to the front of the Shine-Dalgarno sequence of the gene encoding the polypeptide to form a secondary structure incapable of ribosome attachment may make mRNA translation impossible or reduce the translation rate.

The 8) addition of a promoter transcribed in the reverse direction to the 3' terminus of an open reading frame (ORF) of the gene sequence encoding the polypeptide (Reverse transcription engineering, RTE) may be to weaken the activity by making an antisense nucleotide complementary to the transcript of the gene encoding the polypeptide.

The 9) regulation of cellular localization of a protein (polypeptide) may be to target a protein (polypeptide) to a specific organelle in a cell or a specific intracellular space. For example, it may be to target to the periplasm or cytoplasm through addition or removal of a leader sequence functioning in protein (polypeptide) targeting, but is not limited thereto.

Such weakening of polypeptide activity may be that the activity or concentration expression level of the corresponding polypeptide is weakened based on the activity or concentration of the polypeptide expressed in a wild-type or pre-modification microorganism strain, or the amount of a product produced from the polypeptide is reduced, but is not limited thereto.

In the present disclosure, the term "enhancement" of polypeptide activity means that the activity of the polypeptide is increased compared to the intrinsic activity. The enhancement can be used interchangeably with terms such as activation, up-regulation, overexpression, and increase. Here, activation, enhancement, up-regulation, overexpression, and increase may all include exhibiting an activity that was not originally possessed, or exhibiting an improved activity compared to the intrinsic activity or pre-modification activity. The "intrinsic activity" refers to the activity of a specific polypeptide originally possessed by a parent strain or unmodified microorganism before its trait is changed due to genetic variation caused by natural or artificial factors. This can be used interchangeably with "activity before modification". That the activity of a polypeptide is "enhanced", "up-regulated", "overexpressed" or "increased" compared to the intrinsic activity means that it is improved compared to the activity and/or concentration (expression level) of the specific polypeptide originally possessed by the parent strain or unmodified microorganism before the trait change.

The enhancement can be achieved by introducing an exogenous polypeptide, or through enhancement of endogenous polypeptide activity and/or concentration (expression level). Whether the activity of the polypeptide is enhanced can be confirmed from an increase in the degree of activity of the polypeptide, the expression level, or the amount of a product discharged from the polypeptide.

Enhancement of the activity of the polypeptide can be applied by various methods well known in the art, and is not particularly limited as long as the activity of the target polypeptide can be enhanced more than that of the microorganism before modification. Specifically, it may be using gene engineering and/or protein engineering well known to those skilled in the art, which are routine methods of molecular biology, but is not limited thereto (e.g., Sitnicka et al. Functional Analysis of Genes. Advances in Cell Biology. 2010, Vol. 2. 1-16, Sambrook et al. Molecular Cloning 2012, and the like).

Specifically, the enhancement of the polypeptide of the present disclosure may be by
1) increasing the intracellular copy number of the polynucleotide encoding the polypeptide;
2) replacing a gene expression control region on a chromosome encoding the polypeptide with a sequence having strong activity;
3) modification of an initiation codon of a gene transcript encoding the polypeptide or a nucleotide sequence encoding a 5'-UTR region;
4) modification of amino acid sequence of the polypeptide so that the polypeptide activity is enhanced;
5) modification of a polynucleotide sequence encoding the polypeptide so that the polypeptide activity is enhanced (for example, modification of the polynucleotide sequence of the polypeptide gene so as to code for a polypeptide modified so that the activity of the polypeptide is enhanced);
6) introduction of an exogenous polypeptide exhibiting the activity of the polypeptide or an exogenous polynucleotide encoding the same;
7) codon optimization of the polynucleotide encoding the polypeptide;
8) analyzing the tertiary structure of the polypeptide and selecting an exposed site to modify or chemically modifying it; or
9) regulation of cellular localization of a protein (polypeptide) (cellular localization); or
10) a combination of two or more selected from the above 1) to 9), but is not particularly limited thereto.

The 1) increasing the intracellular copy number of the polynucleotide encoding the polypeptide may be achieved by introducing into a host cell a vector in which the polynucleotide encoding the polypeptide is operably linked and which can replicate and function independently of the host. Alternatively, it may be achieved by introducing one copy or two or more copies of the polynucleotide encoding the polypeptide into the chromosome in the host cell. The introduction into the chromosome can be performed by introducing into the host cell a vector capable of inserting the polynucleotide into the chromosome in the host cell, but is not limited thereto. The vector is as described above.

The 2) replacing the gene expression control region (or expression control sequence) on the chromosome encoding the polypeptide with a sequence having strong activity may be, for example, generating a mutation in the sequence by deletion, insertion, non-conservative or conservative substitution, or a combination thereof so as to further enhance the activity of the expression control region, or replacing it with a sequence having stronger activity. The expression control region is not particularly limited thereto, but may include a promoter, an operator sequence, a sequence encoding a ribosome binding site, and a sequence regulating the termination of transcription and translation. For example, it may be replacing the original promoter with a strong promoter, but is not limited thereto.

Examples of known strong promoters include CJ1 to CJ7 promoters (U.S. Patent No. 7,662,943 B2), lac promoter, trp promoter, trc promoter, tac promoter, lambda phage PR promoter, PL promoter, tet promoter, gapA promoter, SPL7 promoter, SPL13 (sm3) promoter (U.S. Patent No. 10,584,338 B2), O2 promoter (U.S. Patent No. 10,273,491 B2), tkt promoter, yccA promoter, and the like, but are not limited thereto.

The 3) modification of an initiation codon of a gene transcript encoding the polypeptide or a nucleotide sequence encoding a 5'-UTR region may be, for example, substituting with a nucleotide sequence encoding another initiation codon having a higher polypeptide expression rate compared to the endogenous initiation codon, but is not limited thereto.

The modification of the amino acid sequence or polynucleotide sequence in 4) and 5) may be achieved by generating a mutation in the sequence by deletion, insertion, non-conservative or conservative substitution, or a combination thereof in the amino acid sequence of the polypeptide or the polynucleotide sequence encoding the polypeptide so as to enhance the activity of the polypeptide, or by replacing it with an amino acid sequence or polynucleotide sequence improved to have stronger activity or an amino acid sequence or polynucleotide sequence improved to have increased activity, but is not limited thereto. The replacement may specifically be performed by inserting a polynucleotide into a chromosome by homologous recombination, but is not limited thereto. The vector used at this time may further include a selection marker for confirming the chromosomal insertion. The selection marker is as described above.

The 6) introduction of an exogenous polynucleotide exhibiting the activity of the polypeptide may be introduction into a host cell of an exogenous polynucleotide encoding a polypeptide exhibiting the same/similar activity as the polypeptide. The exogenous polynucleotide is not limited in its origin or sequence as long as it exhibits the same/similar activity as the polypeptide. The method used for the introduction can be performed by appropriately selecting a known transformation method by those skilled in the art, and the polypeptide can be produced by expressing the introduced polynucleotide in a host cell, so that its activity can be increased.

The 7) codon optimization of the polynucleotide encoding the polypeptide may be codon optimization so that transcription or translation of an endogenous polynucleotide is increased in a host cell, or codon optimization of an exogenous polynucleotide so that optimized transcription and translation take place in a host cell.

The 8) analyzing the tertiary structure of the polypeptide and selecting an exposed site to modify or chemically modifying it may be, for example, determining a template protein candidate according to the degree of sequence similarity by comparing sequence information of the polypeptide to be analyzed with a database in which sequence information of known proteins is stored, confirming the structure based thereon, and selecting an exposed site to be modified or chemically modified to modify or chemically modify it.

The 9) regulation of cellular localization of a protein (polypeptide) may be to target a protein (polypeptide) to a specific organelle in a cell or a specific intracellular space. For example, it may be to target to the periplasm or cytoplasm through addition or removal of a leader sequence functioning in protein (polypeptide) targeting, but is not limited thereto.

Such enhancement of polypeptide activity may be that the activity or concentration expression level of the corresponding polypeptide is increased based on the activity or concentration of the polypeptide expressed in a wild-type or pre-modification microorganism strain, or the amount of a product produced from the polypeptide is increased, but is not limited thereto.

Modification of part or all of a polynucleotide in the microorganism of the present disclosure (e.g., modification for encoding the above-described type II citrate synthase variant) may be induced by (a) homologous recombination using a vector for chromosomal insertion in a microorganism or genome editing using an engineered nuclease (e.g., CRISPR-Cas9) and/or (b) treatment with light such as ultraviolet rays and radiation and/or chemicals, but is not limited thereto. A method for modifying part or all of the gene may include a method by DNA recombination technology. For example, deletion of part or all of a gene can be achieved by injecting a nucleotide sequence or vector comprising a nucleotide sequence homologous to the target gene into the microorganism to allow homologous recombination to occur. The injected nucleotide sequence or vector may comprise a dominant selection marker, but is not limited thereto.

In the microorganism of the present disclosure, the type II citrate synthase variant, the polynucleotide, the aspartate-derived L-amino acid (e.g., L-lysine), and the like are as described in the other aspects above.

Another aspect of the present disclosure provides a method for producing an aspartate-derived L-amino acid comprising a step of culturing a microorganism of the genus *Corynebacterium* comprising at least one selected from the group consisting of the type II citrate synthase variant of the present disclosure and the polynucleotide encoding the type II citrate synthase variant in a medium.

The method for producing an aspartate-derived L-amino acid of the present disclosure may comprise a step of culturing a microorganism of the genus *Corynebacterium* comprising the type II citrate synthase variant of the present disclosure or the polynucleotide of the present disclosure or the vector of the present disclosure in a medium.

The aspartate-derived L-amino acid is as described in other aspects above.

In the present disclosure, the term "culture" means growing the microorganism of the genus *Corynebacterium* of the present disclosure under appropriately regulated environmental conditions. The culture process of the present disclosure can be carried out according to a suitable culture medium and culture conditions known in the art. Such a culture process can be easily adjusted and used by those skilled in the art according to the selected strain. Specifically, the culture may be batch, continuous and/or fed-batch, but is not limited thereto.

In the present disclosure, the term "culture medium" refers to a substance in which nutrients required for culturing the microorganism of the genus *Corynebacterium* of the present disclosure are mixed as main components, and supplies nutrients and growth factors, including water essential for survival and growth. Specifically, as the culture medium and other culture conditions used for culturing the microorganism of the genus *Corynebacterium* of the present disclosure, any of those used for culturing general microorganisms can be used without particular limitation, but the microorganism of the genus *Corynebacterium* of the present disclosure can be cultured in a common culture medium containing an appropriate carbon source, nitrogen source, phosphorus source, inorganic compounds, amino acids and/or vitamins while regulating temperature, pH, and the like, under aerobic conditions.

Specifically, culture media for a strain of the genus *Corynebacterium* can be found in ["Manual of Methods for General Bacteriology" by the American Society for Bacteriology (Washington D.C., USA, 1981)].

In the present disclosure, the carbon source may include carbohydrates such as glucose, saccharose, lactose, fructose, sucrose, and maltose; sugar alcohols such as mannitol and sorbitol; organic acids such as pyruvic acid, lactic acid, and citric acid; amino acids such as glutamic acid, methionine, and lysine. In addition, natural organic nutrient sources such as starch hydrolysate, molasses, blackstrap molasses, rice bran, cassava, sugarcane residue, and corn steep liquor can be used, specifically carbohydrates such as glucose and sterilized pretreated molasses (i.e., molasses converted to reducing sugar) can be used, and various other appropriate amounts of carbon sources can be used without limitation. These carbon sources can be used alone or in combination of two or more, and are not limited thereto.

As the nitrogen source, inorganic nitrogen sources such as ammonia, ammonium sulfate, ammonium chloride, ammonium acetate, ammonium phosphate, ammonium carbonate, and ammonium nitrate; or organic nitrogen sources such as amino acids such as glutamic acid, methionine, and glutamine, peptone, NZ-amine, meat extract, yeast extract, malt extract, corn steep liquor, casein hydrolysate, fish or decomposition products thereof, defatted soybean cake, decomposition products thereof, or the like, can be used. These nitrogen sources can be used alone or in combination of two or more, and are not limited thereto.

The phosphorus source may include monopotassium phosphate, dipotassium phosphate, or corresponding sodium-containing salts. As inorganic compounds, sodium chloride, calcium chloride, iron chloride, magnesium sulfate, iron sulfate, manganese sulfate, calcium carbonate, and the like can be used, and in addition, amino acids, vitamins, appropriate precursors and/or the like can be included. These components or precursors can be added to the culture medium in a batch or continuous manner. However, it is not limited thereto.

In addition, during the culture of the microorganism of the genus *Corynebacterium* of the present disclosure, the pH of the culture medium may be adjusted by adding compounds such as ammonium hydroxide, potassium hydroxide, ammonia, phosphoric acid, and sulfuric acid to the culture medium in an appropriate manner. In addition, during culture, an antifoaming agent such as fatty acid polyglycol ester can be used to inhibit bubble formation. In addition, in order to maintain the aerobic condition of the culture medium, oxygen or an oxygen-containing gas can be injected into the culture medium, or to maintain anaerobic and microaerobic conditions, gas injection can be omitted or nitrogen, hydrogen or carbon dioxide gas can be injected, and it is not limited thereto. In one embodiment, the method for producing an aspartate-derived L-amino acid (e.g., L-lysine) may be performed at pH 6 to pH 9, and more specifically, can be performed at pH 6 to pH 9, pH 6 to pH 8.5, pH 6 to pH 8, pH 6 to pH 7.5, pH 6 to pH 7.25, pH 6 to pH 7, pH 6.5 to pH 9, pH 6.5 to pH 8.5, pH 6.5 to pH 8, pH 6.5 to pH 7.5, pH 6.5 to pH 7.25, pH 6.5 to pH 7, pH 6.75 to pH 9, pH 6.75 to pH 8.5, pH 6.75 to pH 8, pH 6.75 to pH 7.5, pH 6.75 to pH 7.25, or pH 6.75 to pH 7, but is not limited thereto.

In the culture of the present disclosure, the culture temperature may be maintained at 20°C to 45°C, specifically 20°C to 45°C, 20°C to 40°C, 20°C to 38°C, 20°C to 37.5°C, 20°C to 37°C, 25°C to 45°C, 25°C to 40°C, 25°C to 38°C, 25°C to 37.5°C, 25°C to 37°C, 30°C to 45°C, 30°C to 40°C, 30°C to 38°C, 30°C to 37.5°C, 30°C to 37°C, 32.5°C to 45°C, 32.5°C to 40°C, 32.5°C to 38°C, 32.5°C to 37.5°C, 32.5°C to 37°C, 35°C to 45°C, 35°C to 40°C, 35°C to 38°C, 35°C to 37.5°C, or 35°C to 37°C, but is not limited thereto. In the culture of the present disclosure, the culture time may be for about 10 to 160 hours, but is not limited thereto.

The aspartate-derived L-amino acid (e.g., L-lysine) produced by the culture of the present disclosure may be secreted into the culture medium or remain in the cell.

The method for producing an aspartate-derived L-amino acid (e.g., L-lysine) of the present disclosure may further include a step of preparing the microorganism of the genus *Corynebacterium* of the present disclosure, a step of preparing a culture medium for culturing the strain, or a combination thereof (in any order), for example, before the culturing step.

The method for producing an aspartate-derived L-amino acid (e.g., L-lysine) of the present disclosure may further include a recovering step of recovering the aspartate-derived L-amino acid (e.g., L-lysine) from the culture medium (culture medium in which culture was performed) or the microorganism of the genus *Corynebacterium* according to the culture. The recovering step may be further included after the culturing step.

The recovery may be to collect the target L-amino acid using a suitable method known in the art according to the culture method of the microorganism of the present disclosure, for example, a batch, continuous, or fed-batch culture method. For example, centrifugation, filtration, crystallization, treatment with a protein precipitant (salting out), extraction, ultrasonic disruption, ultrafiltration, dialysis, or various chromatographies such as molecular sieve chromatography (gel filtration), adsorption chromatography, ion exchange chromatography, and affinity chromatography, HPLC, or a combination of thereof may be used, and the target L-amino acid can be recovered from the culture medium or microorganism using a suitable method known in the art.

In addition, the method for producing an aspartate-derived L-amino acid (e.g., L-lysine) of the present disclosure may additionally comprise a step of purifying. The purification may be performed using a suitable method known in the art. In one embodiment, when the method for producing an aspartate-derived L-amino acid (e.g., L-lysine) of the present disclosure comprises both a step of recovering and a step of purifying, the step of recovering and the step of purifying may be performed continuously or discontinuously regardless of order, or may be performed simultaneously or integrated into one step, but is not limited thereto.

In the method of the present disclosure, variants, polynucleotides, aspartate-derived L-amino acids, vectors, strains, and the like are as described in the other aspects above.

Another aspect of the present disclosure provides a composition for producing an aspartate-derived L-amino acid comprising at least one selected from the group consisting of the type II citrate synthase variant of the present disclosure, the polynucleotide encoding the type II citrate synthase variant of the present disclosure, a vector comprising the polynucleotide, the above-described microorganism of the present disclosure, and a culture medium in which the microorganism is cultured.

The composition of the present disclosure may further comprise any suitable excipient commonly used in a composition for producing an amino acid, and such excipient may be, for example, a preservative, a wetting agent, a dispersing agent, a suspending agent, a buffering agent, a stabilizer, an isotonic agent, or the like, but is not limited thereto.

In the composition of the present disclosure, variants, polynucleotides, vectors, strains, culture media, aspartate-derived L-amino acids, and the like are as described in the other aspects above.

According to another aspect of the present disclosure, the present disclosure may provide a method for increasing production ability of an aspartate-derived L-amino acid (e.g., L-lysine) of a microorganism, a method for conferring production ability of an aspartate-derived L-amino acid (e.g., L-lysine) to a microorganism, and/or a method for producing a microorganism with increased aspartate-derived L-amino acid (e.g., L-lysine) production ability, comprising a step of introducing (e.g., transforming) the above-described type II citrate synthase variant of the present disclosure, the polynucleotide encoding the type II citrate synthase variant, and/or the vector comprising the polynucleotide into the microorganism.

In the method for increasing production ability of an aspartate-derived L-amino acid (e.g., L-lysine) of a microorganism, the method for conferring production ability of an aspartate-derived L-amino acid (e.g., L-lysine) to the microorganism, and/or the method for producing a microorganism with increased aspartate-derived L-amino acid (e.g., L-lysine) production ability of the present disclosure, the type II citrate synthase variant, the polynucleotide, the recombinant vector, the aspartate-derived L-amino acid, the microorganism, and the like are as described above.

According to another aspect of the present disclosure, the present disclosure provides a use of one or more selected from the group consisting of the above-described type II citrate synthase variant of the present disclosure; the polynucleotide encoding the type II citrate synthase variant; a recombinant vector comprising the polynucleotide; and a microorganism comprising the type II citrate synthase variant, the polynucleotide encoding the type II citrate synthase variant, and/or the recombinant vector comprising the polynucleotide, for producing an aspartate-derived L-amino acid; for producing a microorganism producing an aspartate-derived L-amino acid; and/or for conferring and/or increasing aspartate-derived L-amino acid production ability of a microorganism.

In the use for producing an aspartate-derived L-amino acid; for producing aspartate-derived L-amino acid producing an aspartate-derived L-amino acid; and/or for conferring and/or increasing aspartate-derived L-amino acid production ability of a microorganism, the type II citrate synthase variant, the polynucleotide, the recombinant vector, the aspartate-derived L-amino acid, the microorganism, and the like are as described above.

According to another aspect of the present disclosure, the present disclosure provides a composition, method, product, process, or use characterized by one or more elements disclosed in the present disclosure.

### [ADVANTAGEOUS EFFECTS]

When culturing a *Corynebacterium glutamicum* strain comprising the type II citrate synthase variant of the present disclosure, it is possible to produce an aspartate-derived L-amino acid in a high yield compared to a microorganism having a conventional type II citrate synthase

### [MODE FOR CARRYING OUT THE INVENTION]

Hereinafter, the present application will be described in more detail through examples. These examples are only for explaining the present application in more detail, and it will be obvious to those of ordinary skill in the art that the scope of the present application is not limited by these examples according to the gist of the present application.

### Example

(Throughout the present specification, "%" used to indicate a concentration of a specific substance is (weight/weight) % for solid/solid, (weight/volume) % for solid/liquid, and (volume/volume) % for liquid/liquid unless otherwise stated.)

### Example 1: Derivation of sequence mutation candidates of type II citrate synthase derived from Corynebacterium glutamicum

Mutation candidates were derived through a method of predicting Kcat value through prediction of substrate binding site and corresponding substrate binding site mutation.

For prediction of substrate binding site for regulating type II citrate synthase (GltA) activity, protein structure data such as Protein Data Bank (PDB) format for the amino acid sequence of GltA (SEQ ID NO: 1) was input into the Pyvol program, and a Spherification technique for identifying the shape of the protein as a cluster was implemented to predict the Pocket Site (Ryan H.B. Smith, et al., 2019). Random mutation was performed on the GltA sequence centering on the predicted Pocket Site, and Kcat values of the GltA wild-type sequence and variant sequence were predicted through the following method.

The Kcat value was predicted by generating an artificial intelligence model based on the literature of DLKcat (Feiran Li, et al., 2022). The generation process of the artificial intelligence model is as follows. Based on papers and experimental data, a data set of about 16,838 protein sequences, substrate structure information (Simplified Molecular-Input Line-Entry System, SMILES), and Kcat was secured from the BRENDA enzyme database, which provides information on enzymes such as their function, structure, and reaction mechanism, and SABIO-RK, which provides information on biochemical reactions and their reaction kinetics. Non-quantitative information (protein sequence, and the like) was converted into quantitative information through Convolutional Neural Network (CNN), Graph Neural Network (GNN), or the like, and then an artificial intelligence model was generated by adjusting the weights of matrices and vectors in each step.

As the GltA protein sequence, the wild-type sequence (SEQ ID NO: 1) and the variant sequence predicted as the Pocket site were input, and the KEGG number (C00024) of the substrate acetyl-CoA was input to obtain the predicted Kcat value, and among them, the following 4 variants having a Kcat value lower than that of the GltA wild-type sequence were secured: a variant in which the 272nd leucine (L) from the N-terminus in the amino acid sequence of SEQ ID NO: 1 is substituted with arginine (R) (L272R, SEQ ID NO: 3); a variant in which the 310th arginine (R) from the N-terminus in the amino acid sequence of SEQ ID NO: 1 is substituted with tyrosine (Y) (R310Y, SEQ ID NO: 4), a variant in which the 238th histidine (H) from the N-terminus in the amino acid sequence of SEQ ID NO: 1 is substituted with isoleucine (I) (H238I, SEQ ID NO: 7); and a variant in which the 393rd phenylalanine (F) from the N-terminus in the amino acid sequence of SEQ ID NO: 1 is substituted with tyrosine (Y) (F393Y, SEQ ID NO: 8).

### Example 2: Vector construction comprising type II citrate synthase variant sequence and introduction into strain

To construct a vector comprising each predicted mutation in GltA, primers were prepared based on the wild-type gltA gene sequence of SEQ ID NO: 2. To construct a vector comprising the GltA(H238I) sequence, primers of SEQ ID NOs: 19 and 22 and primers of SEQ ID NOs: 20 and 21 were used; to construct a vector comprising the GltA(L272R) sequence, primers of SEQ ID NOs: 19 and 24 and primers of SEQ ID NOs: 20 and 23 were used; to construct a vector comprising the GltA(R310Y) sequence, primers of SEQ ID NOs: 19 and 26 and primers of SEQ ID NOs: 20 and 25 were used; and to construct a vector comprising the GltA(F393Y) sequence, primers of SEQ ID NOs: 19 and 27 and primers of SEQ ID NOs: 20 and 28 were used as pairs, and template PCR was performed using the chromosome of *Corynebacterium glutamicum* ATCC13032 as a template. PCR conditions were performed by repeating 30 cycles of denaturation at 95°C for 30 seconds, annealing at 55°C for 30 seconds, and polymerization at 72°C for 1 minute 30 seconds after denaturation at 95°C for 5 minutes, and then performing a polymerization reaction at 72°C for 5 minutes. Each template PCR fragment was fusion-cloned into plasmid pDC24 (Korean Patent Publication No. 10-2024-0167588, SEQ ID NOs: 29 of the publication) for insertion and replacement of a gene in the Corynebacterium chromosome after treatment with restriction enzymes BamHI and XbaI at 37°C for 4 hours. An In-Fusion^{®} HD cloning kit (Clontech) was used, and after that, transformation was performed into *E. coli* DH5α and spread on LB solid medium containing kanamycin (25 mg/l). A vector in which a target gene was inserted was confirmed through PCR using SEQ ID NOs: 29 and 30, and a final target plasmid was obtained by confirming the sequence of the target gene. Each plasmid was named pDC_gltA(H238I), pDC_gltA(L272R), pDC_gltA(R310Y), and pDC_gltA(F393Y). The sequences of the primers used in this example are shown in Table 1 below.

**[Table 1]**

| SEQ ID NO: | Sequence (5' →3') |
|---|---|
| 19 | GCTCGGTACCCGGGGATCCATGTTTGAAAGGGATATCG |
| 20 | CTGCAGGTCGACTCTAGACATTTCGCTGGCGCGCGAGCT |
| 21 | GCACGCTGACATCGAGCAGAACTGCTCCACCT |
| 22 | CAGTTCTGCTCGATGTCAGCGTGCAGGATGAG |
| 23 | GTCCGGCCCACGGCACGGTGGCGCAAACCAGG |
| 24 | CGCCACCGTGCCGTGGGCCGGACAGAGCGTTG |
| 25 | AAGACGGCGTCTACCTCATGGGCTTCGGACAC |
| 26 | AAGCCCATGAGGTAGACGCCGTCTTCCTTGT |
| 27 | TCACCGTATTGTACGCAATCGGTCGTCTGCCA |
| 28 | GACCGATTGCGTACAATACGGTGAAGAAGTC |
| 29 | AAGTTGGGTAACGCCAGG |
| 30 | CGGCTCGTATGTTGTGTGG |
| 31 | ACTGATAACAACAAGGCTG |
| 32 | GCTCCTCGCGAGGAACCAA |

The vectors pDC_gltA(H238I), pDC_gltA(L272R), pDC_gltA(R310Y) and pDC_gltA(F393Y) constructed as described above were transformed into *Corynebacterium glutamicum* CJ3P (US 9556463 B2) by an electric pulse method (Van der Rest et al., Appl. Microbiol. Biotechnol. 52:541-545, 1999). After confirming that the mutation of the target gene was introduced using a primer pair of the sequences of SEQ ID NOs: 31 and 32 in a strain in which the target variant was introduced into the gltA gene by homologous recombination, they were named CJ3P_gltA(H238I), CJ3P_gltA(L272R), CJ3P_gltA(R310Y), and CJ3P_gltA(F393Y), respectively.

### Example 3: Comparison of L-lysine production ability of constructed gltA variant strains

With the strains constructed in Example 2 as the experimental group and the parent strain CJ3P as the control group, flask fermentation titer evaluation was conducted to confirm the L-lysine production ability. In addition, for comparison, strains having GltA A169V and N241T mutations known in KR10-2277031B1 and KR10-1915433B1 (CJ3P_gltA(A169V), CJ3P_gltA(N241T)) were also subjected to fermentation titer evaluation under the same conditions.

First, each strain was inoculated into a 250 ml corner-baffled flask containing 25 ml of seed medium, and shake culture was performed at 200 rpm at 37°C for 20 hours. 1 ml of the seed culture solution was inoculated into a 250 ml corner-baffled flask containing 24 ml of production medium, and shake culture was performed at 200 rpm at 37°C for 36 hours. To accurately measure the time of sugar source consumption, 700 µL of the culture solution was taken at 18 hours of culture to measure the L-lysine concentration, and then the production ability of each strain was calculated. The experiment was repeated 3 times, and the average value of the analysis results is shown in Table 2 below. The production ability (g/L/h), production ability improvement (%), and lysine concentration after completion of cultivation (g/L) for each strain experimented are shown in Table 2 below.
<Seed culture medium (pH 7.0)>
   glucose 20 g, peptone 10 g, yeast extract 5 g, urea 1.5 g, KH₂PO₄ 4 g, K₂HPO₄ 8 g, MgSO₄·7H2O 0.5 g, biotin 0.1 mg, thiamine HCl 1 mg, calcium pantothenate 2 mg, nicotinamide 2 mg (based on 1 liter of distilled water)
<Production culture medium (pH 7.0)>
   glucose 100 g, (NH₄)₂SO₄ 40 g, soy protein 2.5 g, corn steep solids 5 g, urea 3 g, KH₂PO₄ 1 g, MgSO₄ 7H₂O 0.5 g, biotin 0.1 mg, thiamine hydrochloride 1 mg, calcium pantothenate 2 mg, nicotinamide 3 mg, CaCO₃ 30 g (based on 1 liter of distilled water).

**[Table 2]**

| strain | lysine concentration (g/L)) | | lysine production ability (g/L/h) | lysine production ability improvement (%) |
|---|---|---|---|---|
| | 18h | 36h | at 18h | |
| CJ3P (gltA (WT)) | 4.12 | 7.45 | 0.229 | 100 |
| CJ3P_gltA(A169V) | 4.49 | 8.09 | 0.249 | 108.7 |
| CJ3P_gltA(N241T) | 4.66 | 8.12 | 0.259 | 113.1 |
| CJ3P_gltA(H238I) | 2.32 | 3.88 | 0.129 | 56.3 |
| CJ3P_gltA(L272R) | 4.96 | 8.12 | 0.276 | 120.5 |
| CJ3P_gltA(R310Y) | 5.03 | 8.21 | 0.279 | 121.8 |
| CJ3P_gltA(F393Y) | 3.12 | 5.56 | 0.173 | 75.5 |

As shown in Table 2, among the four newly discovered GltA variants, it was confirmed that the two strains CJ3P_gltA(L272R) and CJ3P_gltA(R310Y), comprising GltA(L272R) and gltA(R310Y) variants, respectively, showed 120.5% and 121.8% of L-lysine production ability, respectively, compared to the control group CJ3P, and also showed increased lysine production ability compared to conventionally known variants. Other mutations showed results in which lysine concentration and lysine production ability rather decreased, and particularly in the case of the CJ3P_gltA(H238I) strain, it was confirmed that growth of the strain was completely inhibited.

### Example 4: Construction of effective mutation-integrated strain and confirmation of L-lysine production ability

To construct a strain in which the gltA(N241T) mutation revealed through existing prior literature (Korean Patent No. 10-1915433) and the two novel mutations whose effects were confirmed in Example 3 were integrated and to further confirm the effect, the vectors pDC_gltA(L272R) and pDC_gltA(R310Y) constructed in Example 2 were transformed into C. glutamicum CJ3P_gltA(N241T) by an electric pulse method (Van der Rest et al., Appl. Microbiol. Biotechnol. 52:541-545, 1999). After confirming the strains in which mutations were introduced into the gltA gene by homologous recombination using a primer pair of SEQ ID NO: 23 and 24, they were named CJ3P_gltA(N241T/L272R) and CJ3P_gltA(N241T/R310Y).

With the strains obtained through this as the experimental group and CJ3P_gltA(N241T) as the control group, flask fermentation titer evaluation was performed in the same manner as in Example 3 to confirm the L-lysine production ability.

**[Table 3]**

| Strain | lysine concentration (g/L) | | lysine production ability (g/L/h) | lysine production ability improvement (%) |
|---|---|---|---|---|
| | 18h | 36h | at 18h | |
| CJ3P_gltA(N241T) | 4.71 | 8.15 | 0.262 | 100 |
| CJ3P_gltA(N241T/L272R) | 5.35 | 8.35 | 0.297 | 113.4 |
| CJ3P_gltA(N241T/R310Y) | 5.46 | 8.43 | 0.303 | 115.6 |

As shown in Table 3, it was confirmed that the two strains CJ3P_gltA(N241T/L272R) and CJ3P_gltA(N241T/R310Y), in which gltA(L272R) and gltA(R310Y) variants were additionally introduced based on the CJ3P_gltA(N241T) strain, had increased L-lysine production ability by 113.4% and 115.6%, respectively, compared to the control strain CJ3P_gltA(N241T).

## Claims

1. A type II citrate synthase variant, wherein an amino acid corresponding to position 272 from the N-terminus, an amino acid corresponding to position 310 from the N-terminus, or a combination thereof in the amino acid sequence of SEQ ID NO: 1 is substituted with another amino acid.

2. The type II citrate synthase variant according to claim 1, wherein the amino acid corresponding to position 272 from the N-terminus is substituted with glutamic acid (E), glycine (G), alanine (A), serine (S), threonine (T), cysteine (C), valine (V), isoleucine (I), methionine (M), proline (P), phenylalanine (F), tyrosine (Y), tryptophan (W), aspartic acid (D), glutamine (Q), histidine (H), lysine (K), arginine (R), or asparagine (N);
the amino acid corresponding to position 310 from the N-terminus is substituted with glutamic acid (E), glycine (G), alanine (A), serine (S), threonine (T), cysteine (C), valine (V), leucine (L), isoleucine (I), methionine (M), proline (P), phenylalanine (F), tyrosine (Y), tryptophan (W), aspartic acid (D), glutamine (Q), histidine (H), lysine (K), or asparagine (N); or
the amino acid corresponding to position 272 from the N-terminus is substituted with glutamic acid (E), glycine (G), alanine (A), serine (S), threonine (T), cysteine (C), valine (V), isoleucine (I), methionine (M), proline (P), phenylalanine (F), tyrosine (Y), tryptophan (W), aspartic acid (D), glutamine (Q), histidine (H), lysine (K), arginine (R), or asparagine (N), and the amino acid corresponding to position 310 from the N-terminus is substituted with glutamic acid (E), glycine (G), alanine (A), serine (S), threonine (T), cysteine (C), valine (V), leucine (L), isoleucine (I), methionine (M), proline (P), phenylalanine (F), tyrosine (Y), tryptophan (W), aspartic acid (D), glutamine (Q), histidine (H), lysine (K), or asparagine (N).

3. The type II citrate synthase variant according to claim 1, wherein an amino acid corresponding to position 169 from the N-terminus, an amino acid corresponding to position 241 from the N-terminus, or a combination thereof in the amino acid sequence of SEQ ID NO: 1 is additionally substituted with another amino acid.

4. The type II citrate synthase variant according to claim 3, wherein the amino acid corresponding to position 169 from the N-terminus is substituted with glutamic acid (E), glycine (G), serine (S), threonine (T), cysteine (C), valine (V), leucine (L), isoleucine (I), methionine (M), proline (P), phenylalanine (F), tyrosine (Y), tryptophan (W), aspartic acid (D), glutamine (Q), histidine (H), lysine (K), arginine (R), or asparagine (N).

5. The type II citrate synthase variant according to claim 3, wherein the amino acid corresponding to position 241 from the N-terminus is substituted with glutamic acid (E), glycine (G), serine (S), threonine (T), cysteine (C), valine (V), leucine (L), isoleucine (I), methionine (M), proline (P), phenylalanine (F), tyrosine (Y), tryptophan (W), aspartic acid (D), glutamine (Q), histidine (H), lysine (K), arginine (R), or alanine (A).

6. The type II citrate synthase variant according to claim 1, comprising the amino acid sequence of SEQ ID NO: 3 or SEQ ID NO: 4.

7. The type II citrate synthase variant according to claim 3, comprising the amino acid sequence of SEQ ID NO: 9 or SEQ ID NO: 10.

8. The type II citrate synthase variant according to claim 1, wherein the type II citrate synthase variant has an amino acid sequence identity of 80% or more and less than 100% with the amino acid sequence of SEQ ID NO: 1.

9. A polynucleotide encoding the type II citrate synthase variant according to any one of claims 1 to 8.

10. A microorganism of the genus *Corynebacterium* comprising at least one selected from the group consisting of the type II citrate synthase variant according to any one of claims 1 to 8 and the polynucleotide encoding the type II citrate synthase variant.

11. The microorganism according to claim 10, wherein the microorganism of the genus *Corynebacterium* is *Corynebacterium glutamicum*.

12. The microorganism according to claim 10, wherein the microorganism has increased production ability of an aspartate-derived L-amino acid compared to a microorganism of the genus *Corynebacterium* which does not comprise any one selected from the group consisting of the type II citrate synthase variant and the polynucleotide encoding the type II citrate synthase variant.

13. The microorganism according to claim 12, wherein the aspartate-derived L-amino acid is one or more selected from the group consisting of L-aspartate, L-lysine, L-threonine, L-methionine, L-homoserine and L-isoleucine.

14. A composition for producing an aspartate-derived L-amino acid comprising the microorganism of claim 10.

15. A method for producing an aspartate-derived L-amino acid comprising a step of culturing the microorganism of claim 10 in a medium.

16. The method for producing an aspartate-derived L-amino acid according to claim 15, wherein the method further comprises a step of recovering the aspartate-derived L-amino acid from a cultured medium or the microorganism.

17. The method for producing an aspartate-derived L-amino acid according to claim 15, wherein the aspartate-derived L-amino acid is one or more selected from the group consisting of L-aspartate, L-lysine, L-threonine, L-methionine, L-homoserine and L-isoleucine.
